(19) Europäisches Patentamt
European Patent Office
Office européen des brevets

(11) **EP 1 704 820 B1**

(12) **EUROPÄISCHE PATENTSCHRIFT**

(45) Veröffentlichungstag und Bekanntmachung des Hinweises auf die Patenterteilung:
**16.05.2012 Patentblatt 2012/20**

(51) Int Cl.:
***A61B 5/021*** *(2006.01)*

(21) Anmeldenummer: **06001181.4**

(22) Anmeldetag: **20.01.2006**

(54) **Verfahren und Gerät zur nichtinvasiven Blutdruckbestimmung**

Mothod and device for noninvasive determination of blood pressure

Procédé et dispositif de détermination non invasive de la tension artérielle

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **23.03.2005 DE 102005014048**

(43) Veröffentlichungstag der Anmeldung:
**27.09.2006 Patentblatt 2006/39**

(73) Patentinhaber: **Küchler, Gert**
**97236 Randersacker (DE)**

(72) Erfinder: **Küchler, Gert**
**97236 Randersacker (DE)**

(74) Vertreter: **Bauerschmidt, Peter et al**
**Rau, Schneck & Hübner**
**Patentanwälte**
**Königstrasse 2**
**90402 Nürnberg (DE)**

(56) Entgegenhaltungen:
**US-A- 4 807 638 US-A- 5 785 659**
**US-A- 5 865 755 US-B1- 6 511 436**

**Beschreibung**

Die Erfindung betrifft ein Verfahren und ein Gerät zur nichtinvasiven Bestimmung des Blutdrucks eines Patienten. Bei dem Verfahren wird ein elektrisches Messsignal eines Herzstroms und ein Pulsmesssignal einer Pulsdruckwelle, die sich ausgehend vom Herzen innerhalb der Blutgefäße ausbreitet, an einer Pulsmessstelle erfasst sowie aus dem elektrischen Messsignal und dem Pulsmesssignal eine Laufzeit der Pulsdruckwelle zwischen dem Herzen und der Pulsmessstelle ermittelt. Das Gerät umfasst einen EKG-Sensor zur Erfassung eines elektrischen Messsignals eines Herzstroms, einen Pulssensor zur Erfassung eines Pulsmesssignals einer Pulsdruckwelle, die sich ausgehend vom Herzen innerhalb der Blutgefäße bis zu einer Pulsmessstelle ausbreitet, an der der Pulssensor angeordnet ist, und eine Auswerteeinheit zur Ermittlung einer Laufzeit der Pulsdruckwelle zwischen dem Herzen und der Pulsmessstelle aus dem elektrischen Messsignal und dem Pulsmesssignal.

Bekannt ist ein derartiges Verfahren aus der WO 98/25516 A1 und ein derartiges Gerät aus der US 5,709,212. Bei dem bekannten Verfahren wird der Blutdruck als lineare Funktion der ermittelten Laufzeit der Pulsdruckwelle errechnet, bei dem bekannten Gerät dagegen als quadratische Funktion einer Pulsdruckwellengeschwindigkeit, also im Wesentlichen des Kehrwerts der ermittelten Laufzeit.

Auch in der DE 102 49 863 A1 werden ein solches Blutdruckmessverfahren und -gerät beschrieben, wobei allerdings keine Angabe über den verwendeten funktionalen Zusammenhang zwischen ermittelter Laufzeit der Pulsdruckwelle und dem Blutdruck gemacht wird.

Weiterhin werden mit der US 6,599,251 B2 und der DE 100 61 189 A1 Verfahren zur nichtinvasiven Blutdruckbestimmung offenbart, bei denen ein Laufzeitunterschied zwischen zwei an verschiedenen Pulsmessstellen erfassten Pulsdruckwellen ermittelt wird. Für die weitere Auswertung wird ein nichtlinearer Zusammenhang zwischen dem ermittelten Laufzeitunterschied und dem gesuchten Blutdruck verwendet. Genannt sind ein logarithmischer, ein potentieller und ein exponentieller Zusammenhang.

Insgesamt eignen sich diese bekannten Blutdruckmessverfahren und -geräte zwar teilweise besser als herkömmliche, beispielsweise nach dem Riva-Rocci-Prinzip arbeitende Verfahren und Geräte. Ein Aufpumpen einer Armmanschette ist nicht erforderlich, so dass eine damit einhergehende unerwünschte Beeinflussung des Kreislaufs und des zu messenden Blutdrucks vermieden wird. Außerdem wird der Patient weniger gestört. Dennoch liefern die genannten bekannten Blutdruckmessverfahren und -geräte bisweilen zu ungenaue Blutdruckwerte.

Eine Aufgabe der Erfindung besteht nun darin, ein Verfahren der eingangs bezeichneten Art anzugeben, das eine genauere Blutdruckermittlung ermöglicht.

Zur Lösung dieser Aufgabe wird ein Verfahren entsprechend den Merkmalen des Patentanspruchs 1 angegeben.

Erfindungsgemäß werden im Funktionszusammenhang, der bei der Blutdruckermittlung zugrunde gelegt wird, mindestens zwei unterschiedliche Anteile berücksichtigt. Der erste aktive Anteil spiegelt das aktive oder auch kontraktile Elastizitätsverhalten der Blutgefäße wieder. Dieses Verhalten ist durch die Aktion der Myofilamente und durch die Ansteuerung mittels nervaler und humoraler Komponenten bedingt. Der zweite passive Anteil steht dagegen für das passive insbesondere durch das Bindegewebe bedingte Elastizitätsverhalten der Blutgefäße. Insbesondere gehen beide Anteile additiv in den Funktionszusammenhang ein und beschreiben jeweils einen nichtlinearen Zusammenhang zwischen der ermittelten Laufzeit der Pulsdruckwelle und dem Blutdruck. Vorzugsweise umfasst der erste aktive Anteil ein Produkt aus dem Kehrwert der Laufzeit und einem Exponentialausdruck der Laufzeit. Der zweite passive Anteil enthält dagegen bevorzugt einen Potenzausdruck der Laufzeit.

Insgesamt beschreibt der erfindungsgemäße Funktionszusammenhang die tatsächlichen physiologischen Verhältnisse, die die Ausbreitung der Pulsdruckwelle in den Blutgefäßen beherrschen, deutlich besser als die bei den bekannten Verfahren verwendeten Ansätze. Dies liegt an der gezielten und insbesondere gesonderten Berücksichtigung sowohl des kontraktilen als auch des passiven Elastizitätsverhaltens der Blutgefäße. Dadurch werden mit dem erfindungsgemäßen Verfahren deutlich genauere Resultate bei der Blutdruckerfassung erzielt als beim Stand der Technik.

Vorteilhafte Ausgestaltungen des erfindungsgemäßen Verfahrens ergeben sich aus den von Anspruch 1 abhängigen Ansprüchen.

Bei einer Variante wird bei der Blutdruckermittlung ein zusätzlicher Kalibrierungsanteil im Funktionszusammenhang berücksichtigt. Der Kalibrierungsanteil wird insbesondere mittels einer herkömmlichen, insbesondere einer oszillometrischen oder einer nach dem Riva-Rocci-Prinzip durchgeführten Kalibrierungsdruckmessung unter Zuhilfenahme einer aufpumpbaren Manschette ermittelt. Günstig ist, dass eine einzige Kalibrierungsdruckmessung ausreicht. Der Patient muss also nicht wie bei anderen Verfahren zusätzlich in einen zweiten Blutdruckzustand (z.B. Belastungszustand) versetzt werden. Dies ist manchmal nicht ohne Gefährdung des Patienten möglich. Darüber hinaus kann die Kalibrierungsdruckmessung zu einem beliebigen Zeitpunkt, also vor, während oder auch nach der eigentlichen Blutdruckbestimmung, erfolgen. Deren Resultate können auch noch nachträglich mit den Daten aus der Kalibrierungsdruckmessung korrigiert werden. Die Kalibrierungsmessung kann automatisch oder manuell erfolgen.

Vorzugsweise wird das Kalibrierungsblutdrucksignal gleichzeitig mit einem elektrischen Messsignal des Herzstroms und einem Pulsmesssignal der Pulsdruckwelle aufgenommen, wodurch die Genauigkeit der Kalibrierung und damit auch die des später ermittelten Blutdrucks steigt. Aus dem elektrischen Messsignal und dem Pulsmesssignal wird mittels des ersten und des zweiten Teils des Funktionszusammenhangs ein unkalibrierter Blutdruckwert ermittelt, der zusammen mit dem gemessenen Kalibrierungsdrucksignal in den Kalibrierungsanteil des Funktionszusammenhangs eingeht.

Bei einer weiteren vorteilhaften Ausgestaltung wird die Körpergröße des Patienten bei der Blutdruckermittlung berücksichtigt wird. Dadurch steigt die Genauigkeit des Verfahrens. Die von der Pulsdruckwelle zurückgelegte Wegstrecke lässt sich so auf einfache Weise und mit guter Genauigkeit bestimmen. Alternativ oder ergänzend kann auch das Gewicht des Patienten einfließen.

Weiterhin ist es bevorzugt, das elektrische Messsignal und das Pulsmesssignal praktisch am gleichen Ort, also insbesondere unmittelbar benachbart zueinander, zu erfassen. Beispielsweise können der dazu verwendete EKG-Sensor und der Pulssensor in einem gemeinsamen Erfassungsmodul untergebracht sein. Ein derartiges Erfassungsmodul ist insbesondere zur Anbringung an einer Gliedmaße, einem Finger, einer Zehe oder einem Ohr des Patienten bestimmt. Am Patienten sind dann weniger Einzelsensoren anzubringen. Dies ist angenehmer für ihn. Außerdem beugt eine derartig nahe beieinander liegende Erfassung des elektrischen Messsignals und des Pulsmesssignals Messfehlern vor.

Eine weitere Aufgabe der Erfindung besteht darin, ein Gerät der eingangs bezeichneten Art anzugeben, das eine genauere Blutdruckermittlung ermöglicht.

Zur Lösung dieser Aufgabe wird ein Gerät entsprechend den Merkmalen des Patentanspruchs 5 angegeben.

Vorteilhafte Ausgestaltungen des erfindungsgemäßen Geräts ergeben sich aus den von Anspruch 5 abhängigen Ansprüchen. Das erfindungsgemäße Gerät und seine Ausgestaltungen bieten im Wesentlichen die gleichen Vordie bereits im Zusammenhang mit dem erfindungsgemäßen Verfahren und dessen Varianten beschrieben worden sind.

Weitere Merkmale, Vorteile und Einzelheiten der Erfindung ergeben sich aus der nachfolgenden Beschreibung von Ausführungsbeispielen anhand der Zeichnung. Es zeigt:

Fig. 1    ein Ausführungsbeispiel eines Blutdruckmessgeräts zur nichtinvasiven Bestimmung des Blutdrucks eines Patienten in Blockschalt-bilddarstellung,

Fig. 2    ein Ausführungsbeispiel eines im Blutdruckmessgerät gemäß Fig. 1 eingesetzten gemeinsamen Erfassungsmoduls zur Erfassung eines elektrischen Messsignals des Herzstroms und eines Pulsmess-signals einer Pulsdruckwelle,

Fig. 3    im Rahmen des Blutdruckmessgeräts gemäß Fig. 1 erfasste oder abgeleitete Signalverläufe, und

Fig. 4    einen bei der Auswertung im Blutdruckmessgerät gemäß Fig. 1 zugrunde gelegten nichtlinearen Funktionszusammenhang zwischen einer ermittelten Laufzeit einer Pulsdruckwelle und dem gesuchten Blutdruck.

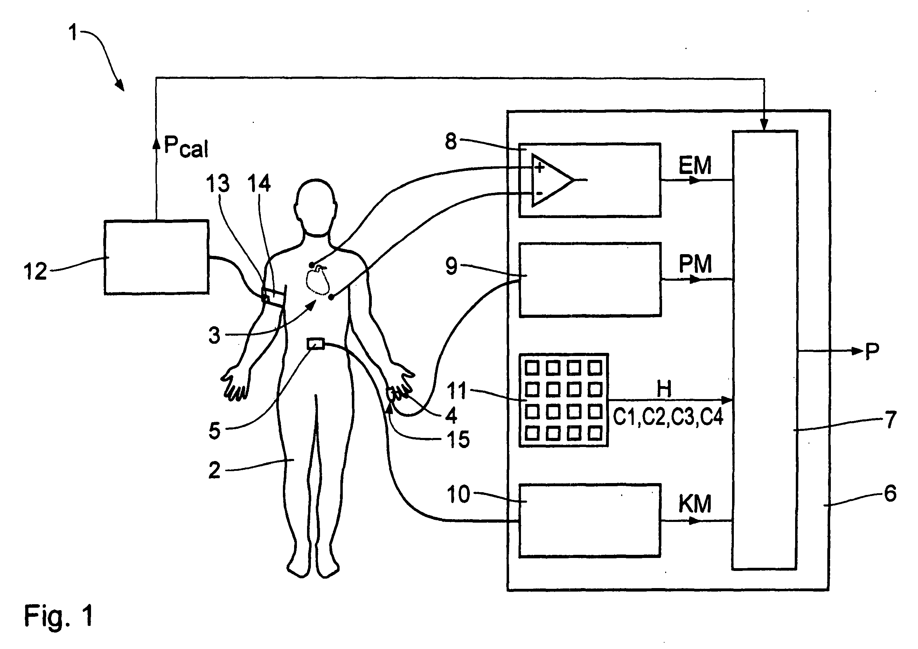

Einander entsprechende Teile sind in den Fig. 1 bis 4 mit denselben Bezugzeichen versehen.

In Fig. 1 ist ein Ausführungsbeispiel eines Blutdruckmessgeräts 1 zur nichtinvasiven Bestimmung des Blutdrucks P eines Patienten 2 gezeigt. Es enthält einen EKG-Sensor 3 mit mindestens zwei Aufnahmeelektroden, einen Pulssensor 4 in Form eines Pulsoximeters sowie einen optionalen Körperlagesensor 5 in Form eines Dreilageschalters, die an eine Auswerteeinheit 6 angeschlossen sind. Die Auswerteeinheit 6 umfasst mehrere Komponenten. Neben einer Berechnungseinheit 7 sind für jeden der angeschlossenen Sensoren spezifische Untereinheiten vorgesehen, also eine EKG-Teileinheit 8, eine Pulsoximeter-Teileinheit 9 sowie eine Körperlage-Teileinheit 10. Diese Komponenten der Auswerteeinheit 6 müssen nicht unbedingt physikalisch getrennt ausgebildet sein. Sie können auch als Unterprogramme einer auf einem Signal- oder Mikroprozessor in der Auswerteeinheit 6 ablaufenden Software realisiert sein. Weiterhin umfasst die Auswerteeinheit 6 Eingabemittel 11.

An die Auswerteeinheit 6 kann zumindest temporär eine Kalibrierungseinheit 12 mit einem herkömmlichen Blutdrucksensor 13 angeschlossen werden. Der Blutdrucksensor 13 ist im Ausführungsbeispiel als Riva-Rocci-Blutdrucksensor mit einer aufpumpbaren Armmanschette 14 ausgebildet. Bei einem anderen nicht gezeigten Ausführungsbeispiel ist die Kalibrierungseinheit 12 ein Bestandteil der Auswerteeinheit 6. Möglich ist ein weiteres Ausführungsbeispiel, bei dem ein Kalibrierungsblutdruckwert $P_{cal}$ über die Eingabemittel 11 in die Auswerteeinheit 6 eingegeben wird.

Im Ausführungsbeispiel gemäß Fig. 1 ist der EKG-Sensor 3 am Brustkorb des Patienten 2 in unmittelbarer Nähe zum Herzen angeordnet. Der Pulssensor 4 ist an einer Pulsmessstelle 15, im Ausführungsbeispiel an einem Finger des Patienten 2, angebracht. Eine andere Pulsmessstelle 15 wie z.B. an einem Ohr, einer Zehe oder einer Gliedmaße ist ebenso möglich. Außerdem kann der Pulssensor 4 anstatt als Pulsoximeter auch als Drucksensor oder als Ultraschallsensor ausgebildet sein.

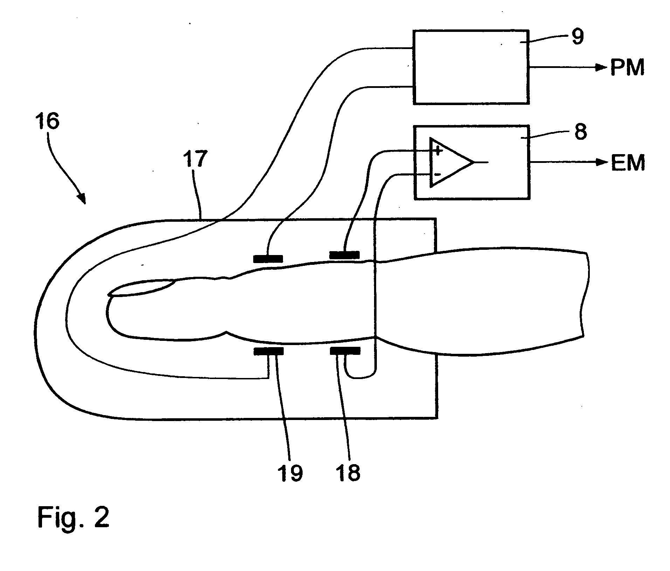

In Fig. 2 ist ein vorteilhaftes Ausführungsbeispiel eines gemeinsamen Erfassungsmoduls 16 gezeigt. In einem als aufschiebbare Fingerkappe ausgeführten Gehäuse 17 sind ein EKG-Sensor 18 und ein Pulssensor 19 untergebracht. Dadurch ist der Platzbedarf besonders gering. Der Pulssensor 19 ist ein Pulsoximeter.

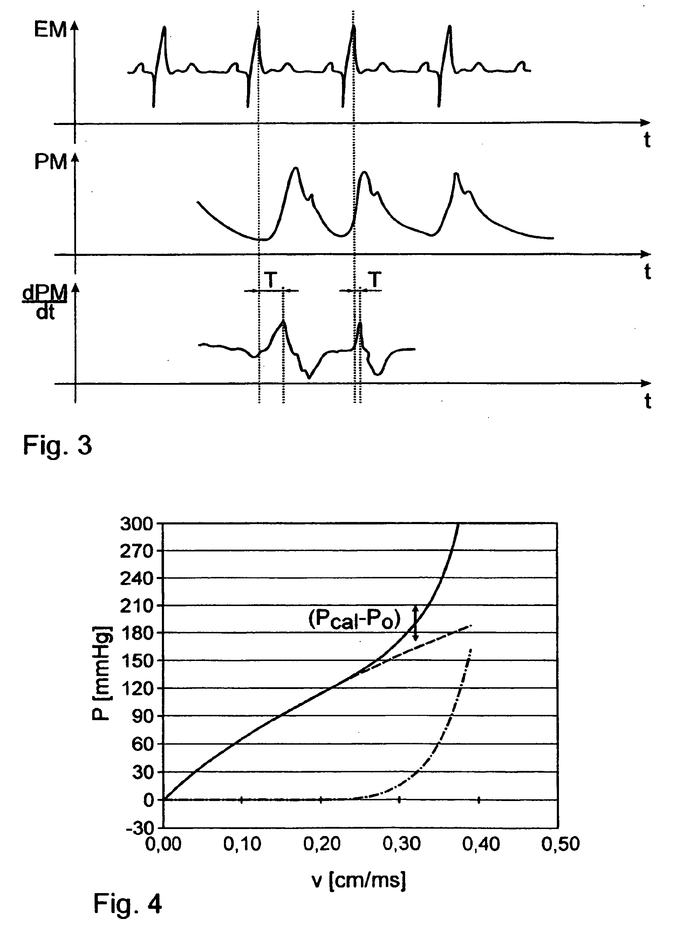

Im Folgenden wird die Funktionsweise des Blutdruckmessgeräts 1 auch unter Bezugnahme auf die in den Fig. 3 und 4 wiedergegebenen Diagramme näher erläutert.

Die EKG-Teileinheit 8 erzeugt aus den von dem EKG-Sensor 3 erfassten Signalen ein elektrisches Messsignal EM (siehe oberes Diagramm von Fig. 3) eines Herzstroms, das zur Weiterverarbeitung in die Berechnungseinheit 7 eingespeist wird. Der Pulssensor 4 erfasst eine an der Pulsmessstelle 15 vorbeilaufende Pulsdruckwelle, die sich ausgehend vom Herzen des Patienten 2 innerhalb der Blutgefäße ausbreitet. Dementsprechend stellt die Pulsoximeter-Teileinheit 9 in Verbindung mit dem Pulssensor 4 der Berechnungseinheit 7 ein Pulsmesssignal PM (siehe mittleres Diagramm von Fig. 3) zur Verfügung. Die Körperlage-Teileinheit 10 liefert in Verbindung mit dem Körperlagesensor 5 ein Körperlagesignal KM an die Berechnungseinheit 7.

In der Auswerteeinheit 6, insbesondere in der Berechnungseinheit 7, wird aus dem elektrischen Messsignal EM und dem Pulsmesssignal PM eine Laufzeit T der Pulsdruckwelle zwischen dem Herzen des Patienten 2 und der Pulsmessstelle 15 ermittelt. Bei Bedarf kann das Pulsmesssignal PM vor dieser Weiterverarbeitung zunächst noch einer Signalaufbereitung unterzogen werden. So kann es insbesondere mittels eines Approximationsverfahrens geglättet oder gefittet werden.

Als Laufzeit T wird die Zeitdifferenz zwischen dem Zeitpunkt der sogenannten R-Zacke im elektrischen Messsignal EM und dem Zeitpunkt der maximalen Steigung im Pulsmesssignal PM verwendet. Zur leichteren Ermittlung des zuletzt genannten Zeitpunkts wird die zeitliche Ableitung des Pulsmesssignals PM gebildet (siehe unteres Diagramm von Fig. 3). Die gesuchte Laufzeit T lässt sich dann einfach durch einen zeitlichen Vergleich der Maximalwerte im Messsignal EM und in der zeitlichen Ableitung des Pulsmesssignals PM bestimmen. In Fig. 3 sind für zwei aufeinanderfolgende Herzschlagzyklen die so bestimmten jeweiligen Laufzeiten T eingetragen.

Aus dieser Laufzeit T wird in der Berechnungseinheit 7 mittels des Funktionszusammenhangs:

$$P = C1 \cdot \frac{D}{T} \cdot e^{C2\frac{D}{T}} + C3 \cdot \left(\frac{D}{T}\right)^{C4} + \left(P_{cal} - P_0\right) =$$
$$= C1 \cdot v \cdot e^{C2 \cdot v} + C3 \cdot v^{C4} + \left(P_{cal} - P_0\right) \qquad (1)$$

ein momentaner systolischer Blutdruck P in [mmHg] errechnet, wobei mit D eine ungefähre Wegstrecke in [cm], die die Pulsdruckwelle zwischen dem Herzen (linker Ventrikel) und der Pulsmessstelle 15 zurücklegt, mit T die Laufzeit der Pulsdruckwelle in [ms], mit C1, C2, C3, C4 jeweils ein vorgegebener oder wählbarer Parameter, mit $P_0$ ein ermittelter unkalibrierter Blutdruckwert in [mmHg], mit $P_{cal}$ ein Kalibrierungsblutdruckwert in [mmHg] und mit v eine Pulsdruckwellengeschwindigkeit in [cm/ms] bezeichnet ist.

Besonders günstig ist ein Parametersatz, bei dem C1 den Wert 700, C2 den Wert (-1), C3 den Wert 766000 und C4 den Wert 9 annimmt. Der Funktionszusammenhang nach Gleichung (1) erhält dann die spezielle Form:

$$P = 700 \cdot \frac{D}{T} \cdot e^{-\frac{D}{T}} + 766000 \cdot \left(\frac{D}{T}\right)^9 + \left(P_{cal} - P_0\right) =$$
$$= 700 \cdot v \cdot e^{-v} + 766000 \cdot v^9 + \left(P_{cal} - P_0\right) \qquad (2)$$

Die Werte der Parameter C1, C2, C3 und C4 sind anhand einer numerischen Optimierung, wie z.B. der Methode der kleinsten Fehlerquadrate, aus einer Testreihe ermittelt worden, bei der die Blutdruckwerte verschiedener Patienten bei verschiedenen definierten Belastungszuständen und in aufrechter Körperposition mit einem herkömmlichen Riva-Rocci-Blutdruckmessgerät erfasst wurden. Alternativ können die Werte für die Parameter C1, C2, C3 und C4 auch an der Auswerteeinheit 6 vor der Blutdruckmessung über die Eingabemittel 11 eingegeben werden.

Die ungefähre Wegstrecke D zwischen dem Herzen und der Pulsmessstelle 15 kann entweder gemessen, abgeschätzt oder aus der über die Eingabemittel 11 eingegebenen Körpergröße H des Patienten gemäß:

$$D = K \cdot H \tag{3}$$

ermittelt werden, wobei K einen für die jeweilige Pulsmessstelle 15 spezifischen Korrelationskoeffizienten bezeichnet. Er nimmt beispielsweise bei einer Pulsmessstelle 15 am Finger eines Erwachsenen einen Wert von etwa 0,5 und bei einer Pulsmessstelle 15 an der Zehe eines Kindes einen Wert von etwa 0,7 an. Grundsätzlich sind neben Gleichung (3) auch andere Berechnungsvorschriften für die ungefähre Wegstrecke D möglich. So kann ergänzend oder alternativ zur Körpergröße H auch das Körpergewicht einfließen.

Der erste Summand in Gleichung (1) oder (2) beschreibt das aktive oder auch kontraktile Elastizitätsverhalten der Blutgefäße, der zweite Summand das passive durch das Bindegewebe bedingte Elastizitätsverhalten der Blutgefäße. Das kontraktile Elastizitätsverhalten ist durch die Aktion der Myofilamente und durch die Ansteuerung mittels nervaler und humoraler Komponenten bedingt. Beide Elastizitäten beeinflussen die Ausbreitung der Pulsdruckwelle innerhalb der Blutgefäße und damit auch die letztlich resultierende Laufzeit T. Beide Einflüsse weichen zum Teil erheblich voneinander ab. Dies wird beim Blutdruckmessgerät 1 durch die verschiedenen algebraischen Ausdrücke in den ersten beiden Summanden der Gleichungen (1) und (2) entsprechend berücksichtigt.

Der dritte Summand in Gleichung (1) oder (2) dient der patientenspezifischen Anpassung. Dieser Kalibrierungsanteil wird mittels einer Kalibrierungsmessung ermittelt, bei der die Kalibrierungseinheit 12 den Kalibrierungsblutdruckwert $P_{cal}$ erfasst und an die Auswerteeinheit 6 weiterleitet. Gleichzeitig mit der Kalibrierungsmessung am herkömmlichen Blutdrucksensor 13 erfolgen Messungen am EKG-Sensor 3 und am Pulssensor 4, aus denen in der Auswerteeinheit 6 die zugehörige Laufzeit T der Pulsdruckwelle und mittels der ersten beiden Summanden der Gleichung (1) oder (2) der unkalibrierte Blutdruckwert $P_0$ ermittelt wird. Eine einzige Kalibrierungsmessung reicht also zur patientenspezifischen Anpassung aus (= Ein-Punkt-Kalibrierung). Zur Kontrolle oder Nachjustierung können natürlich weitere Kalibrierungsmessungen erfolgen.

In dem Diagramm von Fig. 4 ist der erste Summand von Gleichung (2) gestrichelt, der zweite Summand von Gleichung (2) strichpunktiert und die Summe aus beiden mit durchgezogener Linie eingetragen. Aufgetragen ist der Blutdruck P über der Pulsdruckwellengeschwindigkeit v. Der unterschiedliche Einfluss des aktiven und des passiven Anteils wird deutlich. Während der aktive Anteil (= erster Summand) bei niedrigen Pulsdruckwellengeschwindigkeiten v und Blutdrücken P dominiert, überwiegt der passive Anteil (= zweiter Summand) bei hohen Pulsdruckwellengeschwindigkeiten v und Blutdrücken P. Im für die Vielzahl der Anwendungsfälle maßgeblichen mittleren Bereich gehen dagegen beide Anteile ein. Die bekannten Verfahren und Geräte zur Blutdruckmessung werden gerade diesem Einfluss beider Anteile bei weitem nicht in vergleichbarer Weise gerecht. Der dritte Summand von Gleichung (2) bewirkt einen Offset, also eine bezogen auf die v-Achse parallele Verschiebung der Summenkurve (=durchgezogene Linie). Dies ist in Fig. 4 durch den Doppelpfeil angedeutet.

Die so ermittelten Werte für den Blutdruck P werden je nach der mittels des Körperlagesensors 5 erfassten Körperlage korrigiert. Unterschieden wird insbesondere zwischen der aufrechten Körperposition, der Rückenlage, der Bauchlage und der Lage auf der rechten oder linken Körperseite. Dadurch wird der bei diesen Körperpositionen jeweils unterschiedliche hydrostatische Druck kompensiert. Insgesamt ist der Einfluss des mit der Körperlage variierenden hydrostatischen Drucks aber bei dem Blutdruckmessgerät 1 nicht so groß wie bei der herkömmlichen Blutdruckmessung nach dem Riva-Rocci-Prinzip. Dadurch ergibt sich eine leichtere Handhabbarkeit und eine geringere Anfälligkeit für Handhabungsfehler. Außerdem ist es insbesondere bei geringeren Anforderungen an die Messgenauigkeit möglich auf die Erfassung und Einbeziehung der Körperlage zu verzichten.

Die vorstehenden Ausführungen beziehen sich auf die Erfassung eines Messwertes für den systolischen Blutdruck. Der diastolische Blutdruck lässt sich aber grundsätzlich nach dem gleichen Prinzip erfassen.

Der mit dem Blutdruckmessgerät 1 ermittelte Blutdruck P weist einen hohen Korrelationskoeffizienten von mindestens 0,82 verglichen mit Werten, die mittels eines herkömmlichen Riva-Rocci-Blutdruckmessgeräts aufgenommen werden. Das Blutdruckmessgerät 1 eignet sich darüber hinaus sehr gut zur Durchführung einer Langzeit-Blutdruckmessung, die beispielsweise über einen Zeitraum von 24 Stunden erfolgt. Ein weiterer Vorteil besteht darin, dass der Blutdruck P praktisch kontinuierlich, also von Herzschlag zu Herzschlag erfasst werden kann. Dies ist bei herkömmlichen Riva-Rocci-Blutdruckmessgeräten nicht möglich.

**Patentansprüche**

1. Verfahren zur nichtinvasiven Bestimmung des Blutdrucks (P) eines Patienten (2), bei dem

    a) ein elektrisches Messsignal (EM) eines Herzstroms erfasst wird,
    b) ein Pulsmesssignal (PM) einer Pulsdruckwelle, die sich ausgehend vom Herzen innerhalb der Blutgefäße ausbreitet, an einer Pulsmessstelle (15) erfasst wird,
c) aus dem elektrischen Messsignal (EM) und dem Pulsmesssignal (PM) eine Laufzeit (T) der Pulsdruckwelle zwischen dem Herzen und der Pulsmessstelle (15) ermittelt wird, **dadurch gekennzeichnet, dass**
d) der Blutdruck (P) mittels eines Funktionszusammenhangs aus der Laufzeit (T) ermittelt wird, wobei der Funktionszusammenhang einen ersten, das aktive Elastizitätsverhalten der Blutgefäße beschreibenden Anteil und einen zweiten, das passive Elastizitätsverhalten der Blutgefäße beschreibenden Anteil umfasst, und für den bei der Blutdruckermittlung zugrunde gelegten Funktionszusammenhang eine Gleichung gemäß:

$$P = C1 \cdot \frac{D}{T} \cdot e^{C2 \cdot \frac{D}{T}} + C3 \cdot \left(\frac{D}{T}\right)^{C4} + \left(P_{cal} - P_0\right)$$

verwendet wird, wobei mit P der zu ermittelnde Blutdruck in [mmHg], mit D eine ungefähre Wegstrecke in [cm], die die Pulsdruckwelle zwischen dem Herzen und der Pulsmessstelle (15) zurücklegt, mit T die Laufzeit der Pulsdruckwelle in [ms], mit C1, C2, C3, C4 jeweils ein vorgegebener oder wählbarer Parameter, mit $P_0$ ein ermittelter unkalibrierter Blutdruckwert in [mmHg] und mit $P_{cal}$ ein Kalibrierungsblutdruckwert in [mmHg] bezeichnet ist.

2. Verfahren nach Anspruch 1, **dadurch gekennzeichnet, dass** bei der Blutdruckermittlung die Körpergröße des Patienten (2) berücksichtigt wird.

3. Verfahren nach Anspruch 1, **dadurch gekennzeichnet, dass** eine Körperlage des Patienten (2) erfasst und bei der Blutdruckermittlung berücksichtigt wird.

4. Verfahren nach Anspruch 1, **dadurch gekennzeichnet, dass** das elektrische Messsignal (EM) und das Pulsmesssignal (PM) praktisch am gleichen Ort erfasst werden.

5. Gerät zur nichtinvasiven Bestimmung des Blutdrucks (P) eines Patienten (2) umfassend

a) einen EKG-Sensor (3; 18) zur Erfassung eines elektrischen Messsignals (EM) eines Herzstroms,
b) einen Pulssensor (4; 19) zur Erfassung eines Pulsmesssignals (PM) einer Pulsdruckwelle, die sich ausgehend vom Herzen innerhalb der Blutgefäße bis zu einer Pulsmessstelle (15) ausbreitet, an der der Pulssensor (4; 19) angeordnet ist,
c) eine Auswerteeinheit (6) zur Ermittlung einer Laufzeit (T) der Pulsdruckwelle zwischen dem Herzen und der Pulsmessstelle (15) aus dem elektrischen Messsignal (EM) und dem Pulsmesssignal (PM), **dadurch gekennzeichnet, dass**
d) die Auswerteeinheit (6) ausgelegt ist zur Ermittlung des Blutdrucks (P) aus der Laufzeit (T) mittels eines Funktionszusammenhangs, der einen ersten, das aktive Elastizitätsverhalten der Blutgefäße beschreibenden Anteil und einen zweiten, das passive Elastizitätsverhalten der Blutgefäße beschreibenden Anteil umfasst, und in der Auswerteeinheit (6) für den Funktionszusammenhang zur Blutdruckermittlung eine Gleichung gemäß:

$$P = C1 \cdot \frac{D}{T} \cdot e^{C2 \cdot \frac{D}{T}} + C3 \cdot \left(\frac{D}{T}\right)^{C4} + \left(P_{cal} - P_0\right)$$

hinterlegt ist, wobei mit P der zu ermittelnde Blutdruck in [mmHg], mit D eine ungefähre Wegstrecke in [cm], die die Pulsdruckwelle zwischen dem Herzen und der Pulsmessstelle (15) zurücklegt, mit T die Laufzeit der Pulsdruckwelle in [ms], mit C1, C2, C3, C4 jeweils ein vorgegebener oder wählbarer Parameter, mit $P_0$ ein ermittelter unkalibrierter Blutdruckwert in [mmHg] und mit $P_{cal}$ ein Kalibrierungsblutdruckwert in [mmHg] bezeichnet ist.

6. Gerät nach Anspruch 5, **dadurch gekennzeichnet, dass** Mittel (11) zur Eingabe oder Erfassung der Körpergröße (H) des Patienten (2) vorgesehen sind.

7. Gerät nach Anspruch 5, **dadurch gekennzeichnet, dass** ein an die Auswerteeinheit (6) angeschlossener Körperlagesensor (5) zur Erfassung der Körperlage des Patienten (2) vorgesehen ist.

8. Gerät nach Anspruch 5, **dadurch gekennzeichnet, dass** der EKG-Sensor (18) und der Pulssensor (19) in einem gemeinsamen Erfassungsmodul (16) untergebracht sind.

**Claims**

1. A method of noninvasively determining a blood pressure (P) of a patient (2), in which

   a) an electrical cardiac potential measurement signal (EM) is detected;
   b) a pulse measurement signal (PM) of a pulse pressure wave which, starting from the heart, propagates within the blood vessels is detected at a pulse-feeling location (15);
   c) a propagating time (T) of the pulse pressure wave between the heart and the pulse-feeling location (15) is determined, based on the electrical measurement signal (EM) and the pulse measurement signal (PM);
   **characterized in that**
   d) the blood pressure (P) is determined from the propagating time (T) by means of a function interrelationship, wherein the function interrelationship comprises a first portion specifying an active elasticity behaviour of the blood vessels and a second portion specifying a passive elasticity behaviour of the blood vessels, and in the function interrelationship the blood pressure determination is based on, use is made of an equation

$$P = C1 \cdot \frac{D}{T} \cdot e^{C2 \cdot \frac{D}{T}} + C3 \cdot \left(\frac{D}{T}\right)^{C4} + \left(P_{cal} - P_0\right)$$

   with P being a to-be-determined blood pressure in [mmHg]; with D being an approximate distance in [cm] which the pulse pressure wave covers between the heart and the pulse-feeling location (15); with T being the propagating time of the pulse pressure wave in [ms]; with C1, C2, C3, C4 being a given or selectable parameter; with $P_0$ being a determined, uncalibrated blood pressure value in [mmHg]; and with $P_{cal}$ being a calibration blood pressure value in [mmHg].

2. A method according to claim 1, **characterized in that** a body height of the patient (2) is considered in determining the blood pressure.

3. A method according to claim 1, **characterized in that** a body position of the patient (2) is detected and considered in determining the blood pressure.

4. A method according to claim 1, **characterized in that** the electrical measurement signal (EM) and the pulse measurement signal (PM) are detected virtually at the same location.

5. An apparatus for noninvasively determining of the blood pressure (P) of a patient (2), comprising

   a) an ECG sensor (3; 18) for detecting an electrical cardiac potential measurement signal (EM);
   b) a pulse sensor (4; 19) for detecting a pulse measurement signal (PM) of a pulse pressure wave which, starting from the heart, propagates within the blood vessels as far as to a pulse-feeling location (15) where the pulse sensor (4; 19) is disposed;
   c) an evaluation unit (6) for determining a propagation time (T) of the pulse pressure wave between the heart and the pulse-feeling location (15) on the basis of the electrical measurement signal (EM) and the pulse measurement signal (PM);
   **characterized in that**
   d) the evaluation unit (6) is designed for determining the blood pressure (P) from the propagating time (T) by means of a function interrelationship which comprises a first portion that specifies the active elasticity behaviour of the blood vessels, and a second portion that specifies the passive elasticity behaviour of the blood vessels, an equation for the blood pressure determination function interrelationship is deposited in the evaluation unit (6), according to which

$$P = C1 \cdot \frac{D}{T} \cdot e^{C2\frac{D}{T}} + C3 \cdot \left(\frac{D}{T}\right)^{C4} + \left(P_{cal} - P_0\right)$$

with P being the to-be-determined blood pressure in [mmHg]; with D being an approximate distance in [cm] which the pulse pressure wave covers between the heart and the pulse-feeling location (15); with T being the propagating time of the pulse pressure wave in [ms]; with C1, C2, C3, C4 being a given or selectable parameter; with $P_0$ being a determined, uncalibrated blood pressure value in [mmHg]; and with $P_{cal}$ being a calibration blood pressure value in [mmHg].

6. An apparatus according to claim 5, **characterized in that** means (11) are provided for input or detection of the body height (H) of the patient (2).

7. An apparatus according to claim 5, **characterized in that** a body-position sensor (5) connected to the evaluation unit (6) is provided for determining the body position of the patient (2)

8. An apparatus according to claim 5, **characterized in that** the ECG sensor (18) and the pulse sensor (19) are accommodated in a joint detection module (16).

**Revendications**

1. Procédé de détermination non invasive de la tension artérielle (P) d'un patient (2), dans lequel

   a) un signal de mesure électrique (EM) d'un courant cardiaque est décelé,
   b) un signal de mesure du pouls (PM) d'une onde de pression artérielle qui se propage dans les vaisseaux sanguins en partant du coeur, est déterminé au niveau d'un point de mesure du pouls (15),
   c) une période (T) de l'onde de la pression artérielle entre le coeur et le point de mesure du pouls (15) est déterminée à partir du signal de mesure électrique (EM) et du signal de mesure du pouls (PM),
   **caractérisé en ce que**
   d) la tension artérielle (P) est déterminée à partir de la période (T) au moyen d'une correspondance fonctionnelle, la correspondance fonctionnelle comprenant une première partie décrivant le comportement en élasticité active des vaisseaux sanguins et une seconde partie décrivant le comportement en élasticité passive des vaisseaux sanguins, et pour laquelle on emploie pour la correspondance fonctionnelle établie à la base pour la détermination de la tension artérielle une relation telle que :

$$P = C1 \cdot \frac{D}{T} \cdot e^{C2\frac{D}{T}} + C3 \cdot \left(\frac{D}{T}\right)^{C4} + \left(P_{cal} - P_0\right)$$

où P est la tension artérielle à déterminer en [mm Hg], D est une distance de parcours approximative en [cm] qui est parcourue par l'onde de pulsation entre le coeur et le point de mesure du pouls (15), T est la période de l'onde de pression artérielle en [ms], C1, C2, C3, C4 sont chacun un paramètre prédéterminé ou pouvant être choisi, $P_0$ est une valeur de tension artérielle déterminée sans étalonnage en [mm Hg] et $P_{cal}$ est une valeur de tension artérielle de calibration en [mm Hg].

2. Procédé selon la revendication 1 **caractérisé en ce qu'**on tient compte de la taille corporelle du patient (2) pour la détermination de la tension artérielle.

3. Procédé selon la revendication 1 **caractérisé en ce qu'**on apprécie une position corporelle du patient (2) et qu'on en tienne compte lors de l'évaluation de la tension artérielle.

4. Procédé selon la revendication 1 **caractérisé en ce que** le signal de mesure électrique (EM) et le signal de mesure du pouls (PM) sont réalisés pratiquement au même endroit.

5. Dispositif de détermination non invasive de la tension artérielle (P) d'un patient (2) comprenant

 a) un capteur EKG (3 ; 18) pour l'évaluation d'un signal de mesure électrique (EM) d'un courant cardiaque,
 b) un capteur de pulsation (4 ; 19) pour l'évaluation d'un signal de mesure du pouls (PM) d'une onde de pression artérielle qui se propage à partir du coeur dans les vaisseaux sanguins jusqu'à un point de mesure du pouls (15) où est disposé le capteur de pulsation (4 ; 19),
 c) une unité d'exploitation (6) pour la détermination de la période (T) de l'onde de pression artérielle entre le coeur et le point de mesure du pouls (15) à partir du signal de mesure électrique (EM) et du signal de mesure du pouls (PM),
 **caractérisé en ce que**
 d) l'unité d'exploitation (6) est mise en place pour la détermination de la tension artérielle (P) à partir de la période (T) au moyen d'une correspondance fonctionnelle qui comprend une première partie décrivant le comportement d'élasticité active des vaisseaux sanguins et une seconde partie décrivant le comportement d'élasticité passive des vaisseaux sanguins, et où la correspondance fonctionnelle pour la détermination de la tension artérielle dans l'unité d'exploitation (6) est soumise à une équation telle que :

$$P = C1 \cdot \frac{D}{T} \cdot e^{C2\frac{D}{T}} + C3 \cdot \left(\frac{D}{T}\right)^{C4} + (P_{cal} - P_0)$$

 où P désigne la tension artérielle à déterminer en [mm Hg], D est une distance de parcours approximative en [cm] qui est parcourue par l'onde de pulsation entre le coeur et le point de mesure du pouls (15), T est la période de l'onde de pression artérielle en [ms], C1, C2, C3, C4 sont chacun un paramètre prédéterminé ou pouvant être choisi, $P_0$ est une valeur de tension artérielle déterminée sans étalonnage en [mm Hg] et $P_{cal}$ est une valeur de tension artérielle de calibration en [mm Hg].

6. Dispositif selon la revendication 5 **caractérisé en ce que** des moyens (11) pour l'indication ou l'évaluation de la taille corporelle (H) du patient (2) sont prévus.

7. Dispositif selon la revendication 5 **caractérisé en ce qu'**un capteur de position corporelle (5) relié à l'unité d'exploitation (6) est prévu pour l'appréciation de la position corporelle du patient (2).

8. Dispositif selon la revendication 5 **caractérisé en ce que** le capteur EKG (18) et le capteur de pulsation (19) sont logés dans un module d'évaluation (16) commun.

Fig. 1

EP 1 704 820 B1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9825516 A1
- US 5709212 A
- DE 10249863 A1
- US 6599251 B2
- DE 10061189 A1